# EUROPÄISCHE PATENTSCHRIFT

(11) **EP 0 408 961 B1**
(45) Veröffentlichungstag und Bekanntmachung des Hinweises auf die Patenterteilung: **03.02.1993**
(21) Anmeldenummer: 90112555.9
(22) Anmeldetag: 02.07.1990
(51) Int. Cl.: A61M 16/14

(54) **Kalibrierbare Dosiervorrichtung für ein Gasgemisch**
Calibrating and metering device for a mixture of gases
Dispositif de calibrage et de dosage pour un mélange de gaz

(30) Priorität: 20.07.1989 DE 3924123
(43) Veröffentlichungstag der Anmeldung: 23.01.1991
(73) Patentinhaber: Drägerwerk Aktiengesellschaft, 23542 Lübeck (DE)
(72) Erfinder: Kiske, Siegfried, Dr., D-2401 Gross Grönau (DE); Palm, Ulrich, D-2000 Norderstedt (DE)

(56) Entgegenhaltungen:
- EP-A- 0 264 554
- EP-A- 0 280 846
- DE-A- 2 945 575
- US-A- 4 091 056

## Beschreibung

Die Erfindung betrifft eine Vorrichtung zur Erzeugung und Dosierung eines Gasgemisches, bei der ein Trägergas aus mindestens einer Gasquelle durch eine Versorgungsleitung über ansteuerbare Einstellelemente in eine Verdunstereinheit für die Zugabe eines Fluids in das Trägergas mündet und von dort in eine Verbraucherleitung weitergeführt ist, wobei die Verdunsterleitung mit der Verdunstereinheit durch eine Bypassleitung überbrückbar, und der Trägergasstrom durch Schaltelemente an der Verdunstereinheit vorbeileitbar ist.

Eine derartige Vorrichtung ist in der US-A-4091056 beschrieben.

In diesem Dokument ist eine Vorrichtung zur Verdunstung von Flüssigkeiten beschrieben, bei der ein Trägergasstrom abwechselnd über einen Verdunster oder über eine Umgehungsleitung (Bypassleitung) an dem Verdunster vorbei zu einem Verbraucher geleitet wird. Die Strömung durch die Bypassleitung wird über einen Strömungsmesser geführt, dessen Anzeige bei der dann vorliegenden Strömung auf den Wert Null kalibriert ist. Erst wenn die Trägergasleitung über den Verdunster geschaltet wird, nimmt das Trägergas einen Anteil der verdunsteten Flüssigkeit mit, wodurch sich der Massenstrom vergrößert, und sein Mehranteil wird durch den Druchflußmesser angezeigt.

Die Genauigkeit der angezeigten dosierten Menge an dem Durchflußmesser hängt im wesentlichen auch davon ab, wie genau der Trägergasstrom eingestellt ist. Die Anforderungen an die Genauigkeit der Trägergasdosierung werden bei hohen Volumen- und Massenströmen besser erfüllt als bei sehr geringen Massenströmen, wie z. B. bei einigen Millilitern pro Minute. Diese geringen Massenströme spielen aber insbesondere dann eine Rolle, wenn aus einem Narkosemittelverdunster Narkosemittel in einen geschlossenen Narkosekreislauf eingespeist werden soll. Dabei werden nur solche Mengen an Narkosemittel in das Trägergas eingeführt, wie durch den Verbrauch oder durch mögliche, geringste Leckagen aus dem Atemkreis entnommen worden ist.

Der vorliegenden Erfindung liegt somit die Aufgabe zugrunde, eine Vorrichtung der genannten Art so zu verbessern, daß eine ständige, wiederholbare und entweder von Hand oder automatisch durchgeführte Kalibration der Trägergasdosierung, insbesondere bei geringsten Strömungsmengen, auch ohne Unterbrechung des Dosierbetriebes möglich ist.

Die Lösung der Aufgabe erfolgt dadurch, daß strömungsaufwärts zur Verdunstereinheit eine mit Absperrelementen versehene Kalibrierleitung in ein Kalibriervolumen mündet, an welches eine Füllstandsanzeige angeschlossen ist.

Die Vorteile der Erfindung liegen im wesentlichen darin, daß während eines normalen Schaltzyklus, bei dem das Trägergas durch die Verdunstereinheit geleitet wird, zunächst das Kalibriervolumen bis zu einem gewünschten Druck aufgefüllt wird. Die dafür notwendige Zeit, welche über eine getrennte Zeitmeßvorrichtung erfaßt und der Füllstand des Kalibriervolumens somit angezeigt werden kann, ist ein Maß für die Dosierung des Trägergases. Auf diese Weise kann die eingestellte Dosierung überwacht bzw. korrigiert werden. Die in dem Kalibriervolumen gefangene Trägergasmenge kann nach Öffnen des Absperrelementes in die Verdunstereinheit weitergeleitet und somit das verdunstete Fluid dem Trägergasstrom zugeführt werden, genauso wie es ohne Durchführung einer Kalibrierung möglich ist. Nach eventueller Korrektur der Dosierung durch die ansteuerbaren Einstellelemente werden bei den folgenden Umschaltvorgängen die richtigen Mengen in die zugehörigen Leitungen geführt.

Als Füllstandsanzeiger kann weiterhin ein Druckaufnehmer angebracht sein, der den Inhalt eines in seinen Dimensionen unveränderbaren Kalibriertanks über den Innendruck anzeigt. Wahlweise kann das Kalibriervolumen auch aus einer Kolben-/Zylinder-Einheit bestehen, wobei die Füllstandsanzeige durch einen Hubindikator des Kolbens dargestellt ist.

Die Kalibrierleitung kann entweder als Blindleitung an die Verdunsterleitung angeschlossen und über ein Absperrelement, beispielsweise einem Elektromagnetventil, zu- bzw. abgeschaltet werden, oder die Kalibrierleitung ist Teil der Verdunsterleitung, wobei das Kalibriervolumen im Leitungszug der Verdunsterleitung eingeschlossen ist. Am Ausgang und Eingang des Kalibriervolumens liegt dann jeweils ein Absperrelement, beispielsweise Elektromagnetventile. In beiden Fällen muß die Kalibrierleitung durch Absperrelemente von der Verdunsterleitung abtrennbar sein. Zur Kalibrierung der Trägergasmenge wird dann zunächst die Kalibrierleitung geöffnet und die Weiterführung des Trägergases in die Verdunstereinheit abgesperrt. Nach Befüllung des Kalibriervolumens wird dessen Inhalt entweder in die Verdunstereinheit und von dort der Verbraucherleitung zugeführt, oder er wird zurück in die Kalibrierleitung und über das Schaltelement in der Bypassleitung, von dort der Verbraucherleitung zugeführt, ohne daß es über die Verdunstereinheit geleitet wurde. Auf diese Weise kann gewählt werden, ob der Kalibrierschritt entweder im Zuge einer Zudosierung von Verdunsterflüssigkeit oder im Zuge einer bloßen Zuführung von Trägergas an den Verbraucher durchgeführt werden soll. Die betriebsmäßige Dosierung wird daher durch den Kalibrierschritt nicht eingeschränkt, sondern kann zu beliebigen wählbaren oder automatisch gesteuerten Zeitpunkten zwischengeschaltet werden. Je nach Anzeige des Füllstandsanzeigers kann die Dosierung korrigiert werden.

Ein automatischer Ablauf der Kalibrierung und der eventuellen Korrektur der Dosierung kann dadurch erzielt werden, daß die Einstellelemente als mehrere parallelgeschaltete Anordnungen von überkritisch durchströmten Drosselelementen und Absperrventilen ausgebildet sind, deren Ventilausgänge an einem Sammelpunkt der Versorgungsleitung zusammengefaßt sind, und die von einer Steuereinheit betätigbar sind, an welche weiterhin das Schaltelement, die Absperrelemente und der Füllstandsanzeiger angeschlossen sind. Nach Durchführung eines Kalibrierschrittes kann eine notwendige Korrektur einfach dadurch ausgeführt werden, daß der Füllstandsanzeiger den Istwert an die Steuereinheit abgibt, diese ihn mit dem eingegebenen Sollwert vergleicht und bei entsprechender Abweichung Steuersignale an die Absperrventile gibt und diese in geeigneter Anzahl zu- bzw. abschaltet, um die erforderliche Dosierung einzustellen. Wegen der überkritisch durchströmten Drosselelemente sind die Strömungen bei entsprechender Auslegung unabhängig vom Hinterdruck, und bei entsprechender Wichtung ihrer Durchlaßfähigkeit kann bei geeigneter Auswahl der parallelgeschalteten Drosselelemente die Dosiergenauigkeit mit hinreichender Güte erzielt werden. Besonders einfach ist die Auswahl der zuzuschaltenden Drosselelemente, wenn deren Durchlaßfähigkeit mit einer binären Wichtung versehen ist. Eine notwendige Korrektur der Dosierung kann entweder durch Zuschaltung der erforderlichen parallelgeschalteten Absperrventile erfolgen, oder eine vorgegebene Anzahl von Absperrelementen wird, durch die Steuereinheit gepulst getaktet, wobei die Taktfrequenz und das Pulsbreitenverhältnis durch die Steuereinheit variiert und festgelegt werden können.

Zur Dosierung und Kalibrierung von Gasgemischen kann eine zweite Gasquelle vorgesehen sein, deren zugeordnete Anordnungen von Drosselelementen und Absperrventilen an einen Bypassammelpunkt vereinigt sind, in welchem auch eine erste Gruppe von Drosselelementen und Absperrventilen mündet, die zur ersten Gasquelle gehört. Durch Ansteuerung der entsprechenden Anzahl von Absperrventilen kann in der Bypassleitung ein vorgegebenes Gasgemisch zum Verbraucher gelangen. Soll eine Zudosierung von beispielsweise Narkosemittel aus der Verdunstereinheit zu dem Verbraucher geleitet werden, wird eine zweite Gruppe von Drosselelementen und Absperrventilen, die zur ersten Gasquelle gehören, zu einem Kalibriersammelpunkt geführt, welcher strömungsaufwärts zur Verdunstereinheit in die Kalibrierleitung mündet. Während der Zudosierung fließt somit nur Gas aus der ersten Gasquelle durch die Verdunstereinheit, während durch die Bypassleitung ständig ein Gasgemisch vorgegebener Zusammensetzung strömt. Auch hierbei können sowohl der Trägergasstrom durch die Bypassleitung als auch der Trägergasstrom durch die Verdunstereinheit in das Kalibriervolumen geleitet werden, wenn von beiden Gasquellen die entsprechenden Dosiermengen überprüft werden sollen. Wenn z. B. das Gasgemisch durch die Bypassleitung kalibriert werden soll, wird die Gruppe der Absperrventile und Drosselelemente, welche in den Kalibriersammelpunkt münden, insgesamt abgestellt, so daß bei abgesperrter Bypassleitung und geöffneter Kalibrierleitung das Kalibriervolumen mit dem Gasgemisch gefüllt und dessen Dosierung kontrolliert wird. Dabei ist die Verdunstereinheit durch das ihr vorgeschaltete Absperrelement von der Kalibrierleitung getrennt. Nach erfolgter Kalibrierung des Gasgemisches wird der Inhalt des Kalibriervolumens in die Bypassleitung und zum Verbraucher zurückgeleitet. Soll das für die Zudosierung aus der Verdunstereinheit vorgesehene Trägergas kalibriert werden, welches aus der zweiten Gruppe der Absperrventile und Drosselelemente der ersten Gasquelle zum Kalibriersammelpunkt geführt werden, wird die Bypassleitung über das Absperrelement strömungsaufwärts zum Kalibriervolumen abgetrennt. Das Trägergas füllt das Kalibriervolumen, und die dafür erforderliche Zeit wird gemessen. Nach Abschluß der Kalibrierung und eventuellen Korrektur der Durchströmung durch die Drosselelemente wird der Inhalt des Kalibriervolumens aus der Kalibrierleitung in die Verdunstereinheit geführt, die nunmehr durch das ihr vorgeschaltete Absperrelement mit der Kalibrierleitung verbunden ist.

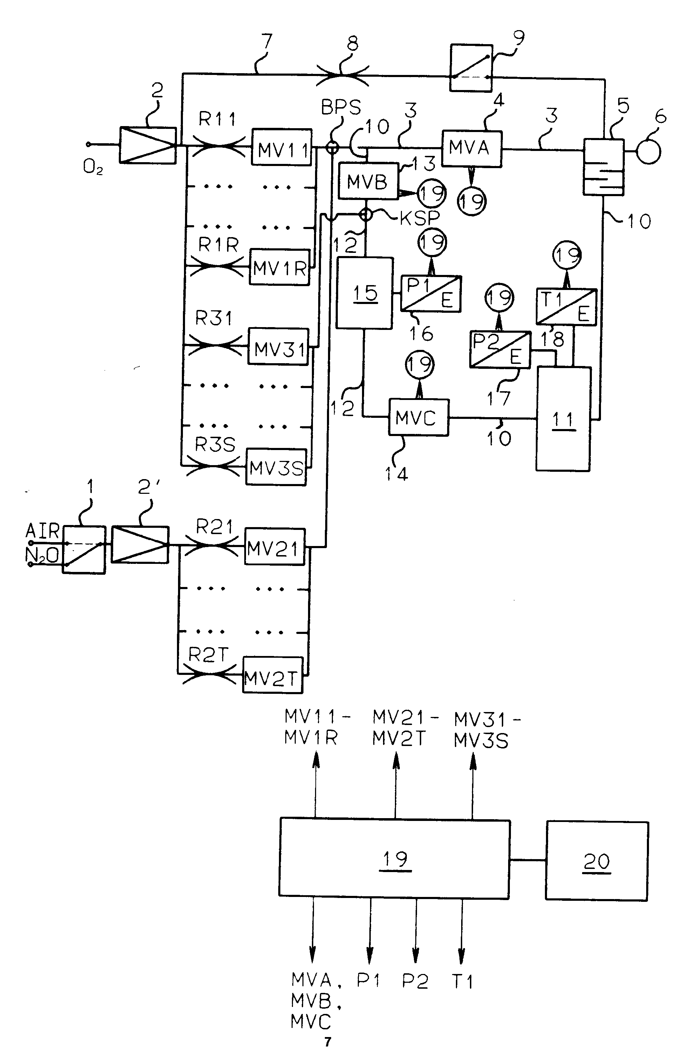

Ein Ausführungsbeispiel der Erfindung wird anhand der Figur schematisch dargestellt und im folgenden näher erläutert.

In der einzigen Figur ist eine Dosier- und Kalibriervorrichtung dargestellt, die aus mindestens zwei verschiedenen Gasquellen, z. B. Sauerstoff (O₂) und Luft (Air) gespeist wird. Dabei ist die zweite Gasquelle über einen Wahlschalter (1) an eine alternative Gasquelle, z. B. N₂O (Lachgas) anschließbar. Der Gasdruck aus den Gasquellen wird über dazugehörige Druckminderer (2, 2′) reduziert. Die erste Gasquelle ist über eine Bypassleitung (3), in welcher sich ein Absperrelement (4) (MVA) befindet, und über eine Mischkammer (5) einem schematisch dargestellten Verbraucher (6) zugeführt. Der Ausgang des Druckminderers (2) ist mit der Mischkammer (5) durch eine Spülleitung (7) verbunden, in der eine Spüldrossel (8) und ein Spülschalter (9) hintereinandergeschaltet sind. Von der Bypassleitung (3) zweigt eine Verdunsterleitung (10) in eine Verdunstereinheit (11) ab, von der aus sie in die Mischkammer (5) geführt ist. Als Teil der Verdunsterleitung (10) ist eine Kalibrierleitung (12) eingesetzt, welche durch Absperrelemente (13, 14) (MVB, MVC) abgegrenzt ist. Zwischen den Absperrelementen (13, 14) ist ein Kalibriervolumen (15) eingesetzt. An das Kalibriervolumen (15) ist ein Druckaufnehmer (16) als Füllstandsanzeiger angeschlossen. Der Druck und die Temperatur in der Verdunstereinheit (11) werden durch Druckfühler (17) und Temperaturfühler (18) aufgenommen. Die erste Gasquelle aus dem Druckminderer (2) wird über eine Vielzahl von parallelgeschalteten Reihenanordnungen von Drosselelementen (R1R, R3S) und Absperrventilen (MV1R, MV3S) geleitet. Von diesen wird die erste Gruppe (MV1R) zu einem Bypassammelpunkt (BPS) in die Bypassleitung (3) zusammengeführt und die zweite Gruppe (MV3S) in einem Kalibriersammelpunkt (KSP) zwischen dem Absperrelement (13) (MVB) und dem Kalibriervolumen (15) zusammengeführt. Der zweiten Gasquelle aus dem Druckminderer (2′) ist eine weitere Parallelschaltung von mehreren Reihenanordnungen von Drosselelementen (R2T) und Absperrventilen (MV2T) nachgeschaltet. Diese werden ebenfalls zu dem Bypassammelpunkt (BPS) geführt. Die einzelnen Absperrventile (MV1R, MV2T, MV3S) sowie die Absperrelemente (4, 13, 14) sind über eine Steuereinheit (19) betätigbar, an welche ebenfalls der Füllstandsanzeiger (16) sowie der Druckfühler (17) und der Temperaturfühler (18) und die Absperrelemente (4, 13, 14) angeschlossen sind. Über eine Eingabeeinheit (20) sind die vom Benutzer vorgesehenen Sollwerte für die Dosierung eingebbar, welche in der Steuereinheit (19) umkodiert werden, so daß sie an die beispielsweise binär kodierten Absperrventile (MV1R, MV2P, MV3S) weitergegeben werden können.

Im Betrieb wird aus der ersten Gasquelle z. B. Sauerstoff in bestimmter Menge durch die Bypassleitung (3) und die Mischkammer (5) dem Verbraucher (6) zugeführt. Durch geeignete Auswahl der auf Durchgang geschalteten Absperrventile (MV1R) und dazugehörigen Drosselelemente (R1R) ist die Gasmenge durch die Bypassleitung (3) bestimmt. Das Absperrelement (4) in der Bypassleitung (3) ist geöffnet und das Absperrelement (13) in der Verdunsterleitung (10) geschlossen. Aus der zweiten Gasquelle wird über den Druckminderer (2′) ein weiteres Gas, z. B. N₂O (Lachgas) zu dem Bypassammelpunkt (BPS) in die Bypassleitung (3) hinzugefügt. Die Menge des Lachgases wird durch die zugeschalteten Absperrventile (MV2T) festgelegt. Der Verbraucher (6) erhält somit eine Mischung aus Sauerstoff und Lachgas, beispielsweise zum Zwecke der Durchführung einer Narkose. Die richtige Dosierung kann durch Einleiten eines Kalibrierschrittes überprüft werden. Hierzu wird das Absperrelement (4) in der Bypassleitung (3) geschlossen, so daß die Strömung durch die Bypassleitung zur Mischkammer (5) unterbrochen ist. Gleichzeitig wird das Absperrelement (13) in der Verdunsterleitung (10) geöffnet, so daß das Gasgemisch vom Bypassammelpunkt (BPS) in das Kalibriervolumen (15) strömt. Das Absperrelement (14) strömungsabwärts zum Kalibriervolumen (15) bleibt geschlossen. Die Füllung des Kalibriervolumens (15) erfolgt bis zu einem vorgegebenen Druckwert, der durch den Füllstandsanzeiger (16) festgelegt ist. Bei Erreichen dieses Druckwertes wird von der Steuereinheit (19) die dafür erforderliche Zeit ermittelt, das Absperrelement (4) geöffnet, so daß der Inhalt des Kalibriervolumens (15) in die Mischkammer (5) zu dem Verbraucher (6) entweichen kann. Aus dem Vergleich der ermittelten Füllzeit des Kalibriervolumens mit dem dafür vorgesehenen Sollwert wird bei Abweichung die entsprechende Anzahl der Absperrventile (MV1R, MV2T) umgeschaltet.

Soll zusätzlich zu dem Sauerstoff-Lachgas-Gemisch ein weiteres Narkosegas aus der Verdunstereinheit (11) in die Mischkammer geführt werden, so ist aus der zweiten Gruppe der parallelgeschalteten Absperrventile (MV3S) Sauerstoff in das Kalibriervolumen (15) zu führen, wobei die Kalibrierleitung (12) durch die Absperrelemente (13, 14) beidseitig des Kalibriervolumens (15) abgeschlossen ist. Das Kalibriervolumen (15) wird so lange gefüllt, bis der für das entsprechende Dosiervolumen erforderliche Fülldruck durch den Füllstandsanzeiger (16) ermittelt und der Steuereinheit (19) übertragen wird. Stimmt das erforderliche Dosiervolumen mit dem eingegebenen Sollwert überein, wird das Absperrelement (14) zwischen Kalibriervolumen (15) und Verdunstereinheit (11) geöffnet, so daß der Inhalt des Kalibriervolumens (15) über die Verdunstereinheit (11) zur Mischkammer geleitet wird. Dabei nimmt die dosierte Menge an Trägergas (im Beispiel Sauerstoff) die sich ergebende verdunstete Menge an Narkosegas mit und führt sie dem Verbraucher (6) zu. Nach Abschluß der erfolgten Zudosierung von zusätzlichem Narkosegas wird das Absperrelement (14) wieder geschlossen und der Füllvorgang des Kalibriervolumens (15) läuft erneut ab. Erst wenn wiederum eine erneute Zudosierung von Narkosemittel über die Kalibrierleitung (12) erforderlich ist, wird das Absperrelement (14) hierzu wieder geöffnet. Sollte auch hierbei die erforderliche Menge zur Füllung des Kalibriervolumens (15) nicht erzielt worden sein, wird durch entsprechende Auswahl der zu öffnenden und zu schließenden Absperrventile (MV3S) durch die Steuereinheit (19) die erforderliche Füllmenge für das Kalibriervolumen (15) eingestellt.

Während der gesamten Zeit der Zudosierung von Narkosegas aus der Verdunstereinheit (11) bleibt das Absperrelement (13) geschlossen und das Narkosegasgemisch, welches an dem Bypassammelpunkt (BPS) anliegt, kann weiterhin durch die Bypassleitung (3) in die Mischkammer gefüllt werden. Dabei wird jedoch diese Menge um den Anteil verringert, der durch die zusätzliche Zuführung von Sauerstoff und verdunstetem Narkosemittel aus der Verdunstereinheit (11) in die Mischkammer hinzugeführt wird. Somit bleibt die Gesamtmenge des dem Verbraucher (6) zugeführten Gases gleich. Statt des Narkosegases N₂O (Lachgas) kann durch den Wahlschalter (1) auch Luft als zweite Gasquelle dem Sauerstoff aus der ersten Gasquelle zugeführt werden.

## Patentansprüche

1. Vorrichtung zur Erzeugung und Dosierung eines Gasgemisches, bei der ein Trägergas aus mindestens einer Gasquelle durch eine Versorgungsleitung über ansteuerbare Einstellelemente in eine Verdunstereinheit (11) für die Zugabe eines Fluids in das Trägergas mündet und von dort zu einem Verbraucher (6) weitergeführt ist, wobei die Verdunsterleitung (10) mit der Verdunstereinheit (11) durch eine Bypassleitung (3) überbrückbar und der Trägergasstrom durch Schaltelemente an der Verdunstereinheit (11) vorbeileitbar ist, dadurch gekennzeichnet, daß strömungsaufwärts zur Verdunstereinheit (11) eine Kalibrierleitung (12) in ein Kalibriervolumen (15) mündet, welches mittels Absperrelementen (13, 14) von der Verdunsterleitung (10) abtrennbar ist, und an welches eine Füllstandsanzeige (16) angeschlossen ist.

2. Vorrichtung nach Anspruch 1, dadurch gekennzeichnet, daß als Füllstandsanzeige ein Druckaufnehmer (16) vorgesehen ist.

3. Vorrichtung nach Anspruch 1 oder 2, dadurch gekennzeichnet, daß zur Kalibrierung der Menge des Trägergasstromes das Schaltelement (4) in der Bypassleitung (3) geschlossen, die Kalibrierleitung (12) in das Kalibriervolumen (15) durch das erste Absperrelement (13) geöffnet und die Weiterführung des Trägergases in die Verdunstereinheit (11) durch das zweite Absperrelement (14) abgesperrt ist, und daß nach Befüllung des Kalibriervolumens (15) dessen Inhalt entweder bei geöffnetem Absperrelement (14) und geschlossenem Absperrelement (13) in die Verdunstereinheit (11) und von dort dem Verbraucher (6), oder bei geschlossenem Absperrelement (14) und geöffnetem Absperrelement (13) und Schaltelement (4) zurück in die Verdunsterleitung (10) und in die Bypassleitung (3) und von dort dem Verbraucher (6) zuführbar ist.

4. Vorrichtung nach einem der Ansprüche 1 bis 3, dadurch gekennzeichnet, daß die Einstellelemente als mehrere parallelgeschaltete Reihenanordnungen von überkritisch durchströmten Drosselelementen (R1R) und Absperrventilen (MV1R) ausgebildet sind, deren Ventilausgänge an einem Bypassammelpunkt (BPS) strömungsaufwärts sowohl der Bypassleitung (3) als auch der Verdunsterleitung (10) zusammengefaßt sind, und die von einer Steuereinheit (19) betätigbar sind, an welche weiterhin das Schaltelement (4), die Absperrelemente (13, 14) und der Füllstandsanzeiger (16) angeschlossen sind.

5. Vorrichtung nach einem der Ansprüche 1 bis 4, dadurch gekennzeichnet, daß eine erste Gasquelle (2) mit ihr zugeordneten Reihenanordnungen von Drosselelementen (R1R, R3S) und Absperrventilen (MV1R, MV3S) vorgesehen ist, von denen eine Gruppe (R13, MV1R) an dem Bypassammelpunkt (BPS) in die Bypassleitung (3) und eine zweite Gruppe (R3S, MV3S) strömungsabwärts des Absperrelementes (13) vor dem Kalibriervolumen (15) an einem Kalibriersammelpunkt (KSP) in die Kalibrierleitung (12) mündet, und daß eine zweite Gasquelle (2′) vorgesehen ist, deren zugeordnete Reihenanordnungen von Drosselelementen (R2T) und Absperrventilen (MV2T) ebenfalls an dem Bypassammelpunkt (BPS) vereinigt sind.

## Claims

1. Device for the generation and dosing of a gas mixture, in which a carrier gas of at least one gas source opens through a supply line by way of controllable adjusting elements into a vaporizer unit (11) for the addition of a liquid into the carrier gas and from here is conducted further to a user (6), whereby the vaporizer line (10) can be bridged with the vaporizer unit (11) by way of a bypass line (3) and the carrier-gas flow can be conducted past the vaporizer unit (11) by way of switching elements, characterized in that upstream of the vaporizer unit (11) a calibrating line (12) opens into a calibrating volume (15), which can be separated from the vaporizer line (10) by means of blocking elements (13, 14), and to which a level display (16) is attached.

2. Device according to claim 1,
characterized in that a pressure sensor (16) is provided as level display.

3. Device according to claim 1 or 2,
characterized in that for the calibration of the quantity of the carrier-gas flow the switching element (4) in the bypass line (3) is closed, the calibrating line (12) into the calibrating volume (15) is opened by means of the first blocking element (13) and the further conduction of the carrier gas into the vaporizer unit (11) is blocked by the second blocking element (14), and that after the filling of the calibrating volume (15) its contents can be supplied either with opened blocking element (14) and closed blocking element (13) into the vaporizer unit (11) and from here to the user (6), or with closed blocking element (14) and opened blocking element (13) and switching element (4) back into the vaporizer line (10) and into the bypass line (3) and from here to the user (6).

4. Device according to one of claims 1 to 3, characterized in that the adjusting elements are constructed as several parallel-connected, tandem-arranged choking elements (R1R) and blocking valves (MV1R) which are flowed through in a supercritical manner, the valve outlets of which are combined at a bypass collecting point (BPS) upstream both of the bypass line (3) and the vaporizer line (10), and which can be actuated by a control unit (19), to which, furthermore, the switching element (4), the blocking elements (13, 14) and the level display (16) are connected.

5. Device according to one of claims 1 to 4, characterized in that a first gas source (2) with tandem-arranged choking elements (R1R, R3S) and blocking valves (MV1R, MV3S) associated therewith is provided, one group (R13, MV1R) of which at the bypass collecting point (BPS) opens into the bypass line (3) and a second group (R3S, MV3S) downstream of the blocking element (13) in front of the calibrating volume (15) at a calibrating collecting point (KSP) opens into the calibrating line (12), and that a second gas source (2′) is provided, whose associated tandem-arranged choking elements (R2T) and blocking valves (MV2T) are likewise united at the bypass collecting point (BPS).

## Revendications

1. Dispositif destiné à préparer et doser un mélange gazeux, dans lequel un gaz porteur arrive, à travers un conduit d'alimentation, par des éléments de réglage commandables, dans une unité d'évaporateur (11) en vue de l'ajout d'un fluide dans le gaz porteur et est acheminé, vers un récepteur (6), le conduit d'évaporateur (10) avec l'unité d'évaporateur (11) pouvant être ponté par un conduit de dérivation (3) et le courant de gaz porteur pouvant être conduit à côté de l'unité d'évaporateur (11), par des éléments de commutation, caractérisé en ce qu'en amont de l'unité d'évaporateur (11), un conduit de calibrage (12) débouche dans un volume de calibrage (15) qui peut être séparé du conduit d'évaporateur (10), au moyen d'éléments d'arrêt (13, 14), et auquel est raccordé un indicateur de niveau (16).

2. Dispositif selon la revendication 1, caractérisé en ce qu'un capteur de pression (16) est prévu comme indicateur de niveau.

3. Dispositif selon la revendication 1 ou 2, caractérisé en ce que pour calibrer la quantité de courant de gaz porteur, l'élément de commutation (4) est fermé dans le conduit de dérivation (3), le conduit de calibrage (12), dans le volume de calibrage (15), est ouvert par le premier élément d'arrêt (13) et l'acheminement du gaz porteur dans l'unité d'évaporateur (11) est bloqué par le second élément d'arrêt (14) et en ce qu'après remplissage du volume de calibrage (15), son contenu peut être acheminé soit, lorsque l'élément d'arrêt (14) est ouvert et l'élément d'arrêt (13) fermé, dans l'unité d'évaporateur (11) et de là vers le récepteur (6), soit, lorsque l'élément d'arrêt (14) est fermé et l'élément d'arrêt (13) ouvert ainsi que l'élément de commutation (14), il peut être renvoyé dans le conduit d'évaporateur (10) ainsi que dans le conduit de dérivation (3) et de là, vers le récepteur (6).

4. Dispositif selon l'une des revendications 1 à 3, caractérisé en ce que les éléments de réglage sont constitués par plusieurs rangées disposées en parallèle d'ensemble d'éléments d'étranglement (R1R), traversés de manière surcritique, et de robinets d'arrêt (MV1R) dont les sorties sont réunies en un point de réunion de dérivation (BPS), en amont du conduit de dérivation (3) ainsi que du conduit d'évaporateur (10), et qui peuvent être actionnés par une unité de commande (19) à laquelle sont raccordés en outre l'élément de commutation (4), les éléments d'arrêt (13, 14) et l'indicateur de niveau (16).

5. Dispositif selon l'une des revendications 1 à 4, caractérisé en ce qu'il est prévu une première source de gaz (2) avec des rangées associées d'ensembles d'éléments d'étranglement (R1R, R3S) et de robinets d'arrêt (MV1R, MV3S) dont un groupe (R13, MV1R) débouche au point de réunion de dérivation (BPS) dans le conduit de dérivation (3) et un second groupe (R3S, MV3S) débouche en aval de l'élément d'arrêt (13), devant le volume de calibrage (15), en un point de réunion de calibrage (KSP), dans le conduit de calibrage (12) et en ce qu'il est prévu une seconde source de gaz (2)′ dont les rangées associées d'ensembles d'élément d'étranglement (R2T) et de robinets d'arrêt (MV2T) sont également réunies au point de réunion de dérivation (BPS).
